# EUROPEAN PATENT APPLICATION

(11) **EP 2 799 535 A1**
(43) Date of publication of application: **05.11.2014**
(21) Application number: 13165688.6
(22) Date of filing: 29.04.2013
(51) Int. Cl.: C12M 1/32, C12M 1/12

(54) **Microstructured membrane for use in a flow cell**

(71) Applicant: FOM Institute for Atomic and Molecular Physics, 1098 XG Amsterdam (NL)
(72) Inventor: Tans, Sander, 1098 XG Amsterdam (NL); Boulineau, Sarah, 1098 XG Amsterdam (NL)
(74) Representative: Visscher, Erik Henk

(57) **Abstract**

Polyacrylamide hydrogel membranes for use in a flow cell device, a flow cell using such membrane and a method for making such membranes are described, said hydrogel membrane comprising a top surface and a bottom surface, wherein one or more microstructures are formed in the bottom surface of said membrane.

## Description

### Field of the invention

The invention relates to membranes for use in a flow cell, and, in particular, though not exclusively, to at least microstructred membrane membrane assembly for use in a flow cell, a flow cell comprising such microstructured membrane and a method of manufacturing such microstructured membrane.

### Background of the invention

The controlled culturing of cells, tissue, and organisms benefits from techniques that combine micron-scale spatial localization, control over the growth media and access for optical imaging and measurement. Spatial localization is important to restrain the moving and moving organisms within the field of view. The ability to create precisely controlled microenvironments has been pursued in microbiology, cell biology and tissue engineering. Currently, there is an interest in integrating such controlled microenvironments with microfluidic techniques that have emerged as an important tool to control transport of chemicals on the micrometer scale and drastically increase the level of parallelization of data acquisition. Fabricating and handling devices that offer such functionality can be challenging, which has limited their use in (commercial) environments without specific microfabrication expertise.

Currently, the most commonly used materials for constructing microstructured flow-cells is the polymer PDMS (polydimethylsiloxane). Flow cells based on PDMS allow for micro-structuring to enclose cells, but PDMS is not porous to aqueous solutions. Hence, it cannot be used to build osmosis or dialysis membranes, and can lead to local medium heterogeneities and accumulation of toxic residues. In addition, PDMS has poorly tunable mechanical properties, which is critical for the correct growth of many cell types. Hence, PDMS does not allow for the establishment of a controlled chemical environment by diffusion. This can be partly circumvented by engineering slits and holes through which only the aqueous media can pass, but this requires complex multilayer designs that are costly, less easy to use, and are limited in the uniformity of the environment.

Instead of PDMS agarose may be used. Agarose is commonly used as an "agar pad", a single monolayer of hydrogel, to confine bacteria, yeast or nematodes to the imaging plane for live microscopy imaging. Simple layers of agarose have also recently been used as membranes to precisely control bacterial medium as a function of time. In addition, agarose may be microstructured to some extent, but the material is mechanically weak and easily breaks when manipulated. Hence is limited in terms of robustness, performance, reuse and design. For example, Moffitt et. al. described in their article "The single-cell chemostat: an agarose-based, microfluidic device for high-throughput, single-cell studies of bacteria and bacterial communities" Lab on Chip, 2012,12,pp.1487-1493 a method for fabricating patterned agarose pads and the use of the patterned agarose pads in the studies of bacteria growth is described.

The patterned agarose pads were fabricated using a silicon master for fabricating a PDMS intermediate master, which is subsequently used for fabricating a patterned agarose pad. According to Moffitt it is not possible to reliably pattern features in the agarose path with aspect ratios (height to width) larger than 0.5. Hence, the control channels, i.e. input and output lines and buffer reservoirs were fabricated in a separate patterned PDMS layer in which the patterned agarose pad was fitted. In addition, agarose is composed of sugars and can be directly metabolized by some organisms or may contain residual non-purified simple sugars, which could interfere with the study of growth under well-controlled conditions.

Hence, there is a need for improved microstructured membranes for use in flow cells. Further, there is a need for improved microstructured membranes that can be used in flow cells, that may be fabricated using a simple process and that allows the fabrication of microstructured membranes comprising microchambers for confining organisms as well as fluidic control channels and/or other functional structures, e.g. a buffer reservoir, that is in diffusive contact with the microchambers.

### Summary of the invention

It is an objective of the invention to reduce or eliminate at least one of the drawbacks known in the prior art. In a first aspect the invention may relate to a polyacrylamide hydrogel membrane, preferably for use in a flow cell, said hydrogel membrane comprising a top surface and a bottom surface, wherein one or more microstructures are formed in the bottom surface of said membrane. The micropatterned molded polyacrylamide membranes are mechanically strong, have good diffusive properties, are transparent to allow for optical access, have low background fluorescence, are low cost, and readily available in many biology labs. The polyacrylamide membranes may be used for building chemostat-like devices for cells and organisms.

In an embodiment said microstructures are molded microstructures. In an embodiment the width and/or depth of each of said one or more microstructures in said bottom surface is selected between 1 and 1000 micron, preferably 2 and 500 micron. In an embodiment, the aspect ratio (height to width) of at least one of said microstructures is larger than 0.5.

In a further aspect, the invention may relate to a olyacrylamide hydrogel membrane assembly, preferably for use in a flow cell device, comprising a polyacrylamide hydrogel membrane as described above on a substrate, wherein the top surface of said substrate and the microstructures in the bottom surface of said hydrogel membrane may form one or more microchambers and/or one or more microchannels.

In an embodiment, said microstructures may be configured such that the lateral distance between a microchamber and a microchannel is selected between 1 and 400 micron, preferably between 2 and 200 micron, more preferably between 4 and 100 micron. The area between the microchamber and the microchannel may be used as a well-defined diffusion barrier.

In an embodiment, said assembly may further comprise a PDMS layer comprising a top and bottom surface, wherein a microchannel is formed in said PDMS layer. The micropatterned molded polyacrylamide membranes can be combined with layers of other materials such as PDMS to construct functionally rich flow-cells in a robust and cheap manner that does not require complex lithography infrastructure.

In yet another embodiment, said assembly may further comprise a cover glass over said polyacrylamide hydrogel membrane, preferably said cover glass isolating said polyacrylamide hydrogel membrane from ambient air.

In yet a further aspect, the invention may relate to a flow cell comprising one or more polyacrylamide hydrogel membranes as described above. The one or more membranes may be positioned between a first substrate and a second substrate, wherein the top surface of said first substrate and the microstructures in the bottom side of said polyacrylamide membrane may form one or more microchambers. Flow-cells based on polyacrylamide membranes allows to maintain delicate living and developing organisms in a manner that is spatially localized, parallelized to many organisms, to maintain constant environments, and to alter the environment in time and space in a controlled manner.

In an embodiment, at least part of said microstructures in said polyacrylamide membrane may form a microchannel, a polyacrylamide region between said microchannel and said one or more microchambers forming a diffusive contact for diffusing liquids, proteins, nutrients, drugs and/or ions in to or out of the microchamber.

In another embodiment, a PDMS layer may be provided over said polyacrylamide membrane, said PDMS comprising one or more microstructures forming a microchannel over said polyacrylamide membrane, a polyacrylamide region between said microchannel and said one or more microchambers forming a diffusive contact for diffusing liquids, proteins, nutrients, drugs and/or ions in to or out of the microchamber.

In yet a further aspect, the invention may relate to a method for forming a molded microstructured polyacrylamide membrane comprising: providing a molding cavity, wherein said cavity comprises one or more microstructures; forming a polyacrylamide hydrogel membrane in said molding cavity by filling the cavity with a solution of acrylamide and a bis-acrylamide cross-linker; removing said polyacrylamide hydrogel membrane from said molding cavity; and, rinsing said polyacrylamide hydrogel membrane for removing non-polymerized acrylamide monomers.

In an embodiment, said ratio between the acrylamide and the bis-acrylamide may be selected between 20:1 and 40:1.

In an embodiment, the width and/or height of said one or more microstructures in said bottom surface is selected between 1 and 1000 micron, preferably 2 and 500 micron; and/or, wherein the aspect ratio (height to width) of at least one of said microstructures is larger than 0.5.

The invention may further relate to the use of microstructures formed in a polyacrylamide hydrogel membrane as described above as culture chambers for bacteria or microorganisms and/or as part of a flow cell.

The physico-chemical properties of polyacrylamide layers are well suited for single cell or organisms studies. In the case of bacteria, spatial confinement and the good affinity between the cell walls and the hydrogel surface favored long term growth in monolayer, which enabled convenient single cell analysis. In the case of yeast cells, soft confinement by the membrane or by microstructures build within the hydrogel ensured localization of the colonies, while maintaining normal growth and morphological phenotypes without requiring the deposition of an additional soft layer on the glass.

The invention may also suitable for the study of larger, multicellular organisms, such as C. elegans. In this context, polyacrylamide gels with microchambers provide two major advantages. First, they allow spatial confinement of these otherwise highly motile organisms, enabling time-lapse microscopy and parallel image acquisition without the use of anesthetic drugs or automated tracking of individual animals. Second, polyacrylamide hydrogels enable exchange of medium and waste products with the microenvironment of the animal.

Compared to the recent use of microfabricated agarose hydrogels, which shares many of these properties, polyacrylamide has the additional advantage of being easier to handle and more resistant to mechanical forces.

Further, the tunable mechanical properties of polyacrylamide hydrogels make them potentially highly useful for mammalian cell culture, because of the exquisite sensitivity of mammalian cells to the mechanical properties of their support. The potential to embed the polyacrylamide membrane in more complex designs offers exciting opportunities to develop precisely-controlled environment for tissue engineering.

The invention will be further illustrated with reference to the attached drawings, which schematically will show embodiments according to the invention. It will be understood that the invention is not in any way restricted to these specific embodiments.

### Brief description of the drawings

**Fig. 1** schematically depicts the fabrication of microstructured polyacrylamide membranes according to an embodiment of the invention.
**Fig. 2** represents images of a substrate comprising a microstructured resist layer and a polyacrylamide membrane in which the microstructured resist layer is moulded.
**Fig. 3A-3C** depict schematics of a microstructured membrane according to various embodiments of the invention.
**Fig. 4A-4D** depicts devices for cells or organisms culture in polyacrylamide membranes according to various embodiments of the invention.
**Fig. 5A-5F** illustrate diffusion in unstructured and structured polyacrylamide hydrogel membranes according to an embodiment of the invention.
**Fig. 6** depicts ... according to an embodiment of the invention.
**Fig. 7A-7C** depicts the use of a microstructured polyacrylamide membrane according to an embodiment of the invention.

### Detailed description

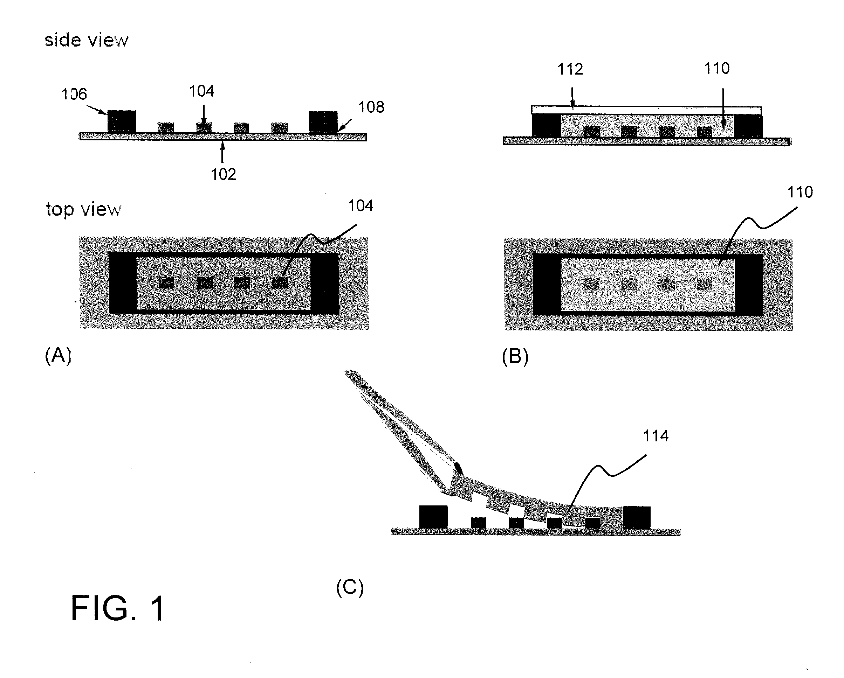

**Fig. 1A-1C** schematically depict the fabrication of microstructured polyacrylamide membranes according to an embodiment of the invention. The polyacrylamide membranes may be produced by soft-lithography using a substrate **102,** e.g. a silicon wafer, comprising the desired micropattern as depicted in **Fig. 1A****.** In an embodiment the micropattern **104** may be made of a resist, e.g. an epoxy resin such as SU-8 epoxy resins of different viscosities resulting in coatings of different thicknesses.

The substrate may be coated, e.g. spin-coated, and standard UV lithography may be used to pattern a predetermined two-dimensional pattern in the coating. Typical dimensions may be selected from a range between 1 and 2000 microns, preferably between 2 and 1000 micron, more preferably between 2 and 500 micron. After UV exposing the coating, the exposed coating may be developed resulting in epoxy microstructures. A confining structure **106,** e.g. a wall structure, may be formed around the microstructured pattern. The confining structure may be made of glass or metal of a height and fixed to the wafer with silicon grease **108** as depicted in **Fig. 1A****.** The height of the confining may be selected between 2 and 10 mm. The confining structure comprising the microstructured pattern may form a molding cavity for the molding process.

Thereafter, molding of the acrylamide may be performed **(****Fig. 1B****).** An aqueous solution **110** of acrylamide monomers may be mixed with curing agents. In an embodiment, a 29:1 ratio of acrylamide / bis-acrylamide (cross-linker) with a final concentration of 10% may be mixed. No additives such as SDS were added to the mixture. The ratio between the acrylamide and the bis-acrylamide may be selected between 20:1 and 40:1 depending on the type of application. This ratio will control the pore size of the hydrogel and thus the mobility of the molecular compounds that may diffuse through the material.

Polymerization may be initiated by the addition of 0,1% of ammonium persulfate (Sigma) and 0.1% of TEMED (Sigma). The mixture may be poured on the master mold within a confining structure and closed with a cover, e.g. a silanized glass coverslip **112.**

A polyacrylamide gel may be obtained after 15-25 min at room temperature. Optimal polymerization may be obtained after 60 through 120 minutes. After polymerization, a micropatterned polyacrylamide membrane **114** is formed wherein one face is shaped as the negative of the micropattern of the molding cavity. Replica of molds below 10 micron down to micron features may be obtained. Unstructured flat polyacrylamide membrane may be obtained using the same process. In an embodiment, silanized glass slide instead of a silicon substrate may be used.

After polymerization, the top coverslip may be removed, after which the gel is cut to specific dimensions, if necessary, and removed with tweezers. In an embodiment, the microstructured polyacrylamide membranes may be rinsed in water to remove non-polymerized toxic acrylamide monomers. In an embodiment, the polyacrylamide may be transferred to fresh purified water at least two times for approximately one hour. This way sufficient biocompatibility was ensured and no apparent growth defects in the organisms studied were observed. The microstructured membrane may be stored for several weeks in an aqueous solution. In an embodiment, before using the polyacrylamide membrane for a cell or organism culture experiment, it may be soaked in an appropriate medium.

While the molding cavity described with reference to **Fig. 1** was fabricated using a simple photolithography technique, any other (micro)fabrication technique may be used to produce a molding cavity comprising microstructures of desired dimensions.

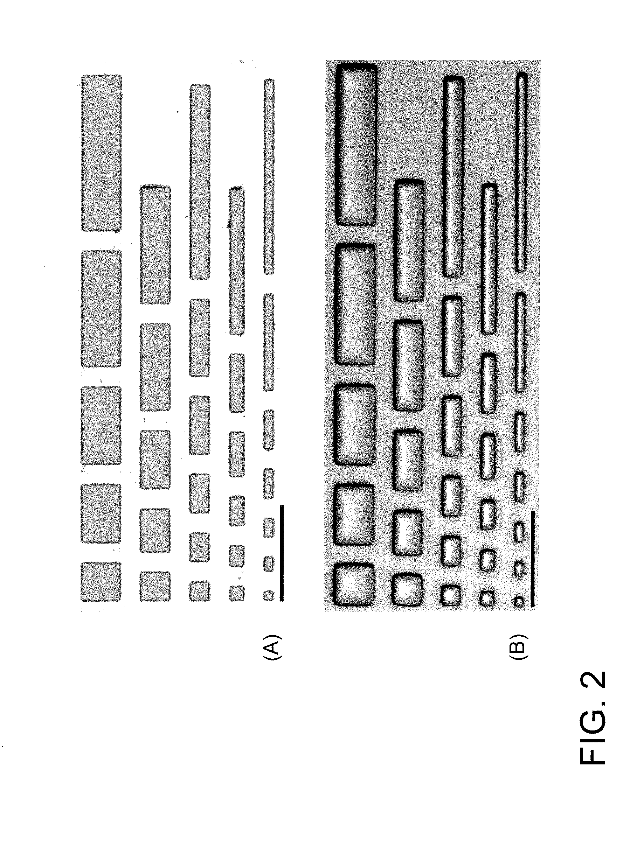

**Fig. 2** represents images of a substrate comprising a microstructured resist layer and a polyacrylamide membrane in which the microstructured resist layer is moulded. The sale bare represents 100 µm. The smallest features in this example are 10 microns. Polyacrylamide gels are mechanically stronger (fracture energy G ∼ 10 - 50 J.m⁻²) than agarose gels (G ∼ 0.1 - 6 J.m⁻²) and hence allow for easier handling and are better suited for microfabrication. The elastic properties of polyacrylamide are tunable over a wide range, which allows to build environments with well controlled mechanical properties. As will be shown hereunder in more detail, polyacrylamide membranes are permeable to aqueous solutions and composed of a synthetic polymer that cannot be metabolized as a carbon source, which allows for excellent control of the growth conditions.

The above-described soft lithography method to transfer micropatterns from a silicon wafer into a polyacrylamide gel allows the integration of confining culture chambers and/or microchannels. In particular, the properties of the polyacrylamide allow a reliable and simple manufacturing process for the realization of membranes comprising microchambers in combination more complex structures such as laterally oriented control channels (micro flow channels) and buffer reservoirs. Fabrication of membranes comprising microchannel structures was previously not possible because of the brittle properties of the agarose which do not allow structures with an aspect ratio (height to width) larger than around 0,5 micron. Furthermore, as will be described in more detail hereunder the polyacrylamide gels may be used as membranes to control the transfer of chemicals, to design devices based on dialysis membranes for changing medium in time or based on diffusion between lateral channels for generating gradients in space.

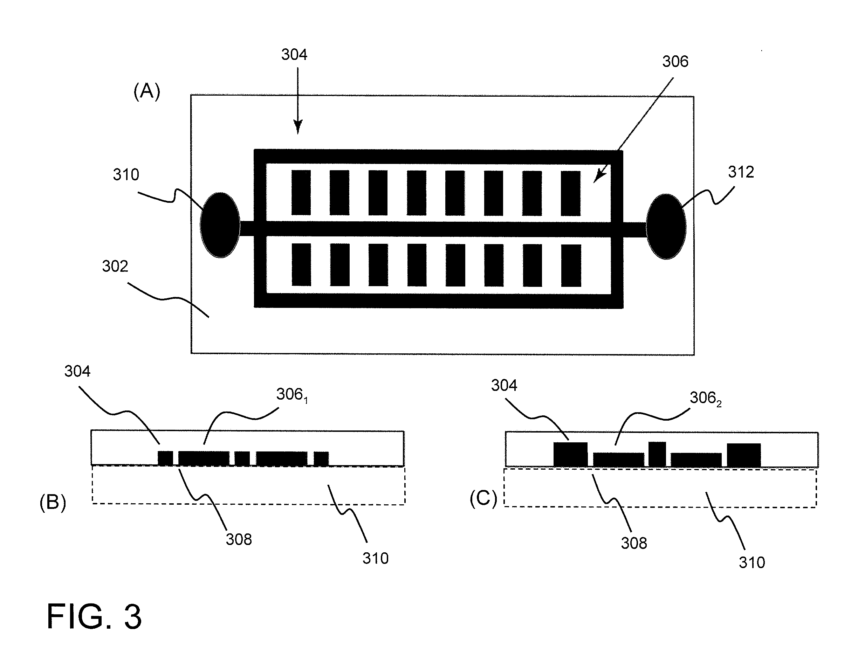

**Fig. 3A-3C** depict schematics of a microstructured membrane according to various embodiments of the invention. In particular, the molding method as described with reference to **Fig. 1** may be used in order to realize a polyacrylamide membrane **302** comprising a molded microfabricated structure as shown in **Fig. 3A** (top view). The microstructure may comprise microstructures that may form microchambers **306** arranged in rows when the microstructured membrane is put in contact with a (glass) substrate **310.** The membrane may further comprise microstructures in its surface that may form microchannels when the membrane is put in contact with a substrate (e.g. a glass substrate). The microchannel microstructures may laterally border the microchamber structures thereby forming an area in the membrane that may function as thin diffusion barrier **308** as shown in **Fig. 3B** (a cross section of the microstructured membrane). Due to the microfabrication, precise control of the width of the diffusion barrier may be obtained. Further, the structure may comprise an inlet reservoir **312** and an outlet reservoir **314.** **Fig. 3C** depicts an alternative embodiment of the microstructured membrane as depicted in **Fig. 3A****.** In this particular embodiment, the molded microstructures (microchambers/control channels) may have different heights and/or widths. In particular, the microstructures that are molded into the polyacrylamide membrane may have different heights and/or widths wherein the structures may have aspect ratios that are larger than 0.5.

It is submitted that the microstructured polyacrylamide membranes are not limited to the examples in this disclosure. Different flow channels associated with different dimensions may be realized (molded) in the membranes. For example, a general flow network may be realized in a membrane comprising microchannels of a width selected between 10 and 400 micron, and a height of 10-100 micron. Similarly, high flow channels may be defined in the membrane comprising microchannels of a width between 200-500 micron and a high between 50 and 200 micron.

Further, micro-chambers for certain cells may be realized for example: micro-chambers for E.Coli baria may have a width between 100 and 300 micron and height between 5 and 50 micron; micro-chambers for yeast may have a width between 200 and 400 micron and height between 10 and 30 micron; and, micro-chambers for mammalian cells may have a width between 200 and 400 micron and height between 20 and 40 micron. Hence, in more general the microchambers that are formed (molded) in the polyacrylamide membrane may have a height and width selected between 1 and 600 micron, preferably between 2 and 500 micron, more preferably between 5 and 400 micron.

Other structures may include buffer reservoirs for control channels, which may have a width selected between 100-500 micron and a height of 50-250 micron and diffusion barriers of large thickness (e.g. a few mm as will be described hereunder in more detail) up to barriers of width between 2 and 200 micron, preferably between 10 micron and 100 micron. Due to the advantageous properties of polyacrylamide, microstructured membranes may be realized comprising microchambers connected via diffusion barriers of a predetermined with to a control channel or a network of control channels and/or reservoirs. As will be shown hereunder in greater detail such structures may be used to controllably deliver drugs via a diffusive barrier to bacteria and/or micro-organisms in a microchamber. These membranes may be reused and can be stored for several weeks in water.

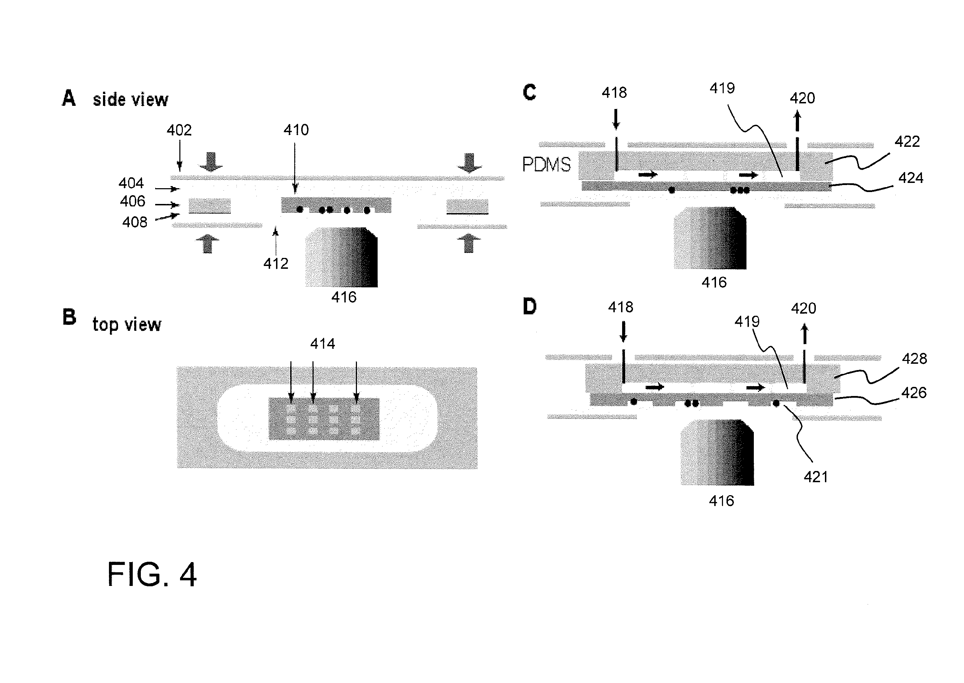

**Fig. 4A-4D** depict devices for cells or organisms culture in polyacrylamide membranes according to various embodiments of the invention. These devices may comprise multiple layers of polyacrylamide and/or PDSM layers which may stacked on top of each other. The entire device may be held together using suitable fastening or claiming means so that the individual gel layers may be re-used. The clamping action may be achieved by a metal holder with screws. Further, the devices are configured with the appropriate openings for microscopy acquisition and microfluidic connectors. The cells or organisms (represented as black circles) may grow at the interface between the polyacrylamide membrane **410** and a glass coverslip **412** such that the cells or organisms at the glass/polyacrylamide interface or in the microchambers may be viewed through the microscope **416.**

**Fig. 4A** depicts a cross-sectional view of a device comprising microfabricated culture chambers in a polyacrylamide membrane according to an embodiment of the invention. In this example, the membrane is enclosed within two glass substrates **404,412** (a glass slide and a glass coverslip) and a sealing structure in the form of surrounding glass spacer **406.** A vacuum grease **408** may be used to realize an airtight seal. The microfabricated polyacrylamide membrane **410** may be mechanically clamped between the two glass substrates. **Fig. 4B** depicts the top view of the device in **Fig. 4A** showing the microchambers **114** in the polyacrylamide membrane surrounded by the sealing structure. On the basis of this simple design sufficient airtightness is ensured to limit evaporation. It may be used for observation of cells or micro-organisms under constant conditions for up to two days, provided that nutrients in the hydrogel membrane are present in large excess.

**Fig. 4C** depicts a cross-section of a flow cell according another embodiment of the invention. In this design, a PDMS layer **422** containing a control channel **419** may be placed on top of and in direct contact with the polyacrylamide membrane **424** thereby allowing continuous diffusion of the medium that runs through the control channel to cells or organisms growing below the hydrogel membrane. The top surface of the PDMS device comprises an inlet **418** and an outlet **420** for accessing the control channel. As no sealing or chemical bonding is required, the PDMS channel can be re-used many times.

Microstructured (molded) polyacrylamide membranes and PDMS control channels may be combined depending on the experimental needs. For example, **Fig. 4D** depicts a device comprising a microstructred polyacrylamide membrane 426 comprising microchambers in combination with a PDMS device comprising a control channel **428** (similar to the one in **Fig. 4C****).**

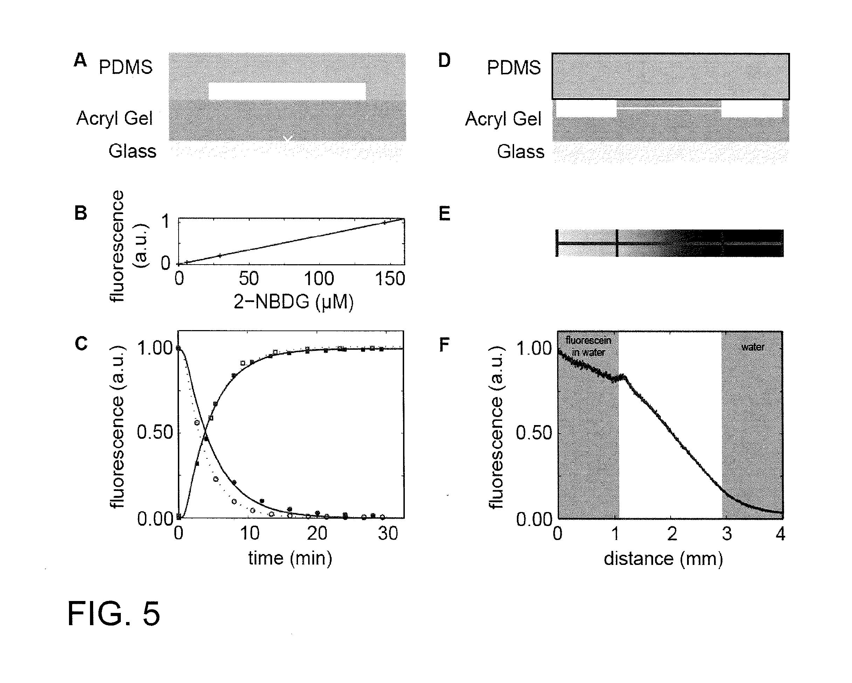

**Fig. 5A-5F** illustrate diffusion in unstructured and structured polyacrylamide hydrogel membranes according to an embodiment of the invention. The measurements illustrate the temporal and spatial control of the microenvironment through the polyacrylamide membranes. The advantageous transport properties of polyacrylamide gels may be shown using a flow cell device as depicted in **Fig. 5A****.** The device comprises unstructured acryl gel (height 500 µm) that is sandwiched between a PDMS layer comprising a channel (height 113 µm), and a glass coverslip (similar to the design in **Fig. 4A****).**

In this experiment, time control of the medium was obtained by placing a 500 µm thick gel membrane between a structured PDMS layer and a glass coverslip (Fig. 5A). Flow was established in the PDMS channel with a syringe pump at flow rates ranging between 10 and 50 µl/min. A fluorescent glucose analog (2-NBDG) was added or removed from the flowing medium at a particular time point by switching a valve, thereby changing the composition of the flowing medium within seconds. The amount of 2-NBDG fluorescence was measured as a function of time by standard fluorescence microscopy using a 100X objective focused on the gel-glass interface.

After the change of medium, the measured fluorescence signal rose in an approximately exponential fashion to the newly imposed steady-state value with a half-time of ∼5 minutes as shown in **Fig. 5B** and **5C****.** Here, **Fig. 5B** shows that the fluorescence of the small dye 2-NBDG is proportional to its concentration in the flowing solution. **Fig. 5C** depicts the fluorescence signal after infusion (squares) or depletion (circles) of the dye 2-NBDG was measured at the gel-glass interface (yellow cross). Lines show fits to the 1D diffusion equation. Open symbols and dashed lines correspond to flow rates of 50 µl/min, closed symbols and solid lines to flow rates of 20 µl/min. Fits to the diffusion profile yielded diffusion coefficients of 4.0x10⁻¹⁰ m²/sec to 5.3x10⁻¹⁰ m²/sec, comparable to the typical diffusion coefficient of small molecules in water (5.0x10⁻¹⁰ m²/sec).

The second experiments aims to set up a spatial concentration gradient by placing a structured gel membrane between a glass slide and a flat PDMS layer, the latter containing inlet and outlet connectors as shown in **Fig. 5D****.**

The linear gradient generator device as shown in **Fig. 5D** comprises a polyacrylamide hydrogel (height 1 mm) that is sandwiched between a PDMS layer and a glass slide. Water containing 3.5 µg/ml fluorescein is flown through the left channel, while pure water is flown through the right channel, thereby creating a linear concentration gradient within the gel. Liquid was pumped through the 100µm high channels molded into the polyacrylamide hydrogel at a rate of 50 µl/min. One channels contained pure water, whereas the other channel contained an aqueous solution of fluorescein molecules.

Diffusion of fluorescein into the polyacrylamide hydrogel, coupled with its removal at the adjacent channel, is predicted to create a linear concentration gradient in the space between the two channels. It was found that the spatial gradient reached steady state after ∼1 hour. Subsequently, the concentration profile was imaged at mid-channel-depth thereby observing a linear concentration gradient within the gel between the two channels as predicted by the theory (see **Fig. 5E** and **5F****).**

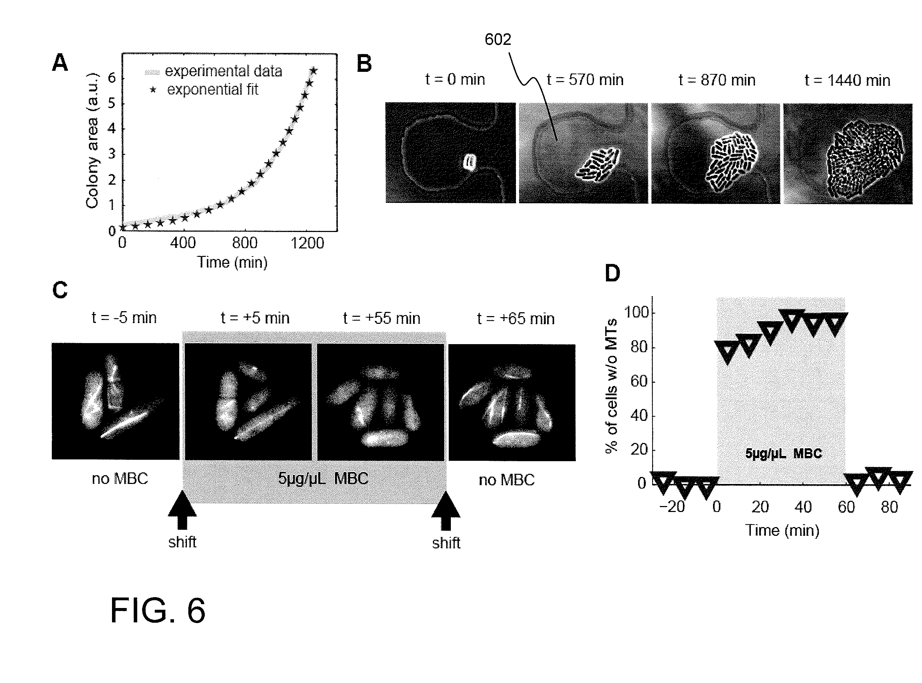

**Fig. 6A-6D** illustrate growth of yeast cells in microchambers in a polyacrylamide membrane and supplying a drug to the yeast cells using a flow cell device according to an embodiment of the invention. In this example, the drug may be supply through the membrane via a control channel that was formed on top of the membrane using a microstructured PDMS layer. First it was checked that the yeasts grows, both confined between an unstructured hydrogel monolayer and a glass cover slip and confined within microstructures such as channels or chambers in a device similar to **Fig. 4A** and **4B****.**

When cells where confined between an unstructured flat hydrogel and glass, a constant exponential proliferation of fission yeast cells for more than 7 generations over 20 hours was observed **(****Fig. 6A****).** This corresponded to an average doubling time of 170 min at 32° C, in agreement with liquid culture growth rate in the same minimal medium. This indicated that the polyacrylamide hydrogel imposed a mechanical pressure sufficient to confine the cells, but was soft enough to not perturb growth, most probably slightly deforming around the cells, which have a 3-4 micron diameter. Yeast cells were grown confined between glass and 3.2 micron deep microstructures that were molded in the hydrogel. **Fig. 6B** depicts images of the microstructure comprising a microchamber **602** for containing the yeast. At one side the microchamber comprises an opening Time lapse imaging showed that colony expansion was constrained by the walls **(****Fig. 6B****).**

The ability of the microstructured membrane combined with a PDMS control channel on top (as shown in **Fig. 4D****)** to control the chemical composition of the microenvironment. In this particular example, microtubule depolymerization was induced during a defined time window. In fission yeast cells, microtubules form 3-5 bundles composed of groups of 2-4 microtubules. These microtubules are involved in multiple biochemical pathways, notably the delivery of polarity factors essential for the control of cell growth.

After growing yeast cells for 2 generations in the microchamber, the flowing medium was supplemented with 50µM of the microtubule-destabilizing drug methyl-2-benzimidazolecarbamate (MBC). Within 5 minutes, fluorescently labeled microtubules disappeared in >80% of the cells **(****Fig. 6C****).** It was observed that in the remaining fraction of the cells, microtubules were organized in mitotic spindles prior to addition of MBC, rendering them more stable. As expected, cell growth was halted in the absence of microtubules. After 60 min, the drug was removed from the flowing medium, leading to the reappearance of microtubules in >90% of cells within 5 min and inducing recovery of the growth process. Hence, the experiment in **Fig. 6A-6D** showed that yeast cells grow normally on polyacrylamide hydrogel membranes, and that colony expansion can be confined between a flat hydrogel membrane and glass or within microfabricated microchambers in the membrane. The membrane proved efficient at adding or removing a drug within minutes.

It is submitted that the invention is not limited to the flow cell devices as used in **Fig. 6A-6D****.** In other embodiments, a drug may be supplied to bacteria and/or micro-organisms in a microchamber of polyacrylamide membrane using a control channel that is microfabricated in the polyacrylamide membrane. In particular, drugs may be supplied to a microchamber using a control channel that is laterally positioned with respect to the microchamber such that a diffusion barrier of a predetermined thickness is realized. An example of such integrated microchamber - control channel structure is schematically depicted in **Fig. 3A-3C.** Using a suitable molding cavity, a polyacrylamide membrane comprising microchambers and integrated control channel structures and reservoirs can be easily fabricated.

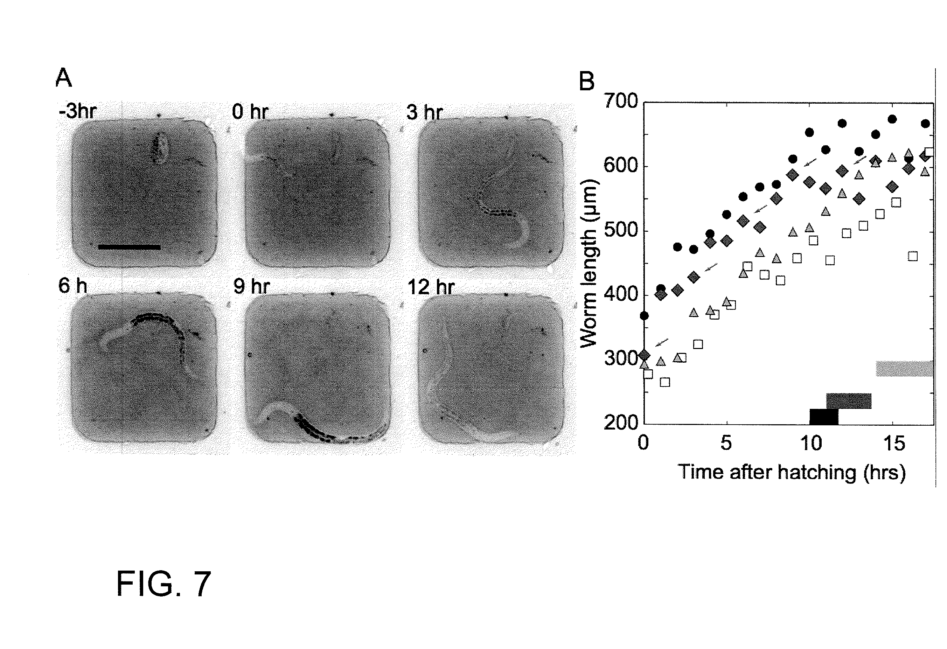

The microstructured polyacrylamide membrane may also be used to confine much larger, multicellular organisms. To that end, in **Fig. 7A** and **7B** the growth and development of single larvae of the nematode worm Caenorhabditis elegans, spatially confined to arrays of microchambers molded into polyacrylamide hydrogel is shown. In particular, **Fig. 7A** depicts images of six stages of the larvae of a single C. elegans animal that is constrained in a 200 x 200 µm polyacrylamide microchamber filled with OP50 as source of food. Time is shown in hours after hatching. At 12 hours after hatching the animal has entered the lethargus at the end of the L1 larval stage. **Fig. 7B** regards a graph indicating the length of the larvae as a function of time after hatching. Different shapes (colors) in the graph are associated with different animals grown in parallel in the microchambers of one device.
C. elegans larvae were grown by adding an individual C. elegans egg and sufficient E. coli bacteria as food source to each microchamber. the eggs developed normally inside the polyacrylamide microchambers and newly hatched larvae increased in length from about ∼300□m directly after hatching to ∼600 □m over the course of 10-15 hrs. All larvae stayed confined to the microchambers during the entire observed period.

The development of C. elegans may be divided in four larval stages, labeled L1 to L4, that are separated by molts during which a new cuticle is synthesized and old cuticle is shed. The molts are accompanied by a behaviorally quiescent state called lethargus. After 10-15 hrs, animals entered the lethargus accompanying the L1 molt **(****Fig. 7B****),** agreeing well with the observed duration of the L1 larval stage of ∼15 hrs. The start of the L1 molt correlated well with animal length. The variability observed in the duration of the L1 stage was mostly due to variation in animal length at the time of hatching. These results indicate that the development of L1 larvae occurred normally inside the microchambers.

It is to be understood that any feature described in relation to any one embodiment may be used alone, or in combination with other features described, and may also be used in combination with one or more features of any other of the embodiments, or any combination of any other of the embodiments. The invention is not limited to the embodiments described above, which may be varied within the scope of the accompanying claims.

## Claims

1. Polyacrylamide hydrogel membrane, preferably for use in a flow cell, said hydrogel membrane comprising a top surface and a bottom surface, wherein one or more microstructures are formed in the bottom surface of said membrane.

2. Hydrogel membrane according to claim 1 wherein said microstructures are molded microstructures.

3. Hydrogel membrane according to claims 1 and 2 wherein the width and/or depth of each of said one or more microstructures in said bottom surface is selected between 1 and 1000 micron, preferably 2 and 500 micron.

4. Hydrogel membrane according to any of claims 1-3 wherein the aspect ratio (height to width) of at least one of said microstructures is larger than 0.5.

5. Polyacrylamide hydrogel membrane assembly, preferably for use in a flow cell device, comprising:
a polyacrylamide hydrogel membrane according to any of claims 1-4 on a substrate, the top surface of said substrate and the microstructures in the bottom surface of said hydrogel membrane forming one or more microchambers and/or one or more microchannels.

6. Membrane assembly according to claim 5 wherein said microstructures are configured such that the lateral distance between a microchamber and a microchannel is selected between 1 and 400 micron, preferably between 2 and 200 micron, more preferably between 4 and 100 micron.

7. Membrane assembly according to claims 5 or 6 further comprising a PDMS layer comprising a top and bottom surface, wherein a microchannel is formed in said PDMS layer.

8. Membrane assembly according to any of claims 5-7 further comprising a cover glass over said polyacrylamide hydrogel membrane, preferably said cover glass isolating said polyacrylamide hydrogel membrane from ambient air.

9. Flow cell comprising one or more polyacrylamide hydrogel membranes according to claims 1-4 wherein said membrane is positioned between a first substrate and a second substrate, the top surface of said first substrate and the microstructures in the bottom side of said hydrogel polyacrylamide membrane forming one or more microchambers.

10. Flow cell according to claim 9 wherein at least part of said microstructures in said polyacrylamide membrane form a microchannel, a polyacrylamide region between said microchannel and said one or more microchambers forming a diffusive contact for diffusing liquids, proteins, nutrients, drugs and/or ions in to or out of the microchamber.

11. Flow cell according to claim 9 wherein a PDMS layer is provided over said polyacrylamide membrane, said PDMS comprising one or more microstructures forming a microchannel over said polyacrylamide membrane, a polyacrylamide region between said microchannel and said one or more microchambers forming a diffusive contact for diffusing liquids, proteins, nutrients, drugs and/or ions in to or out of the microchamber.

12. Method for forming a molded microstructured polyacrylamide membrane comprising:
providing a molding cavity, wherein said cavity comprises one or more microstructures;
forming a polyacrylamide hydrogel membrane in said molding cavity by filling the cavity with a solution of acrylamide and a bis-acrylamide cross-linker;
removing said polyacrylamide hydrogel membrane from said molding cavity; and,
rinsing said polyacrylamide hydrogel membrane for removing non-polymerized acrylamide monomers.

13. Method according to claim 12 wherein said ratio between the acrylamide and the bis-acrylamide may be selected between 20:1 and 40:1.

14. Method according to claims 12 or 13
the width and/or height of said one or more microstructures in said bottom surface is selected between 1 and 1000 micron, preferably 2 and 500 micron; and/or, wherein the aspect ratio (height to width) of at least one of said microstructures is larger than 0.5.

15. Use of microstructures formed in a polyacrylamide hydrogel membrane according to any of claims 1-4 as culture chambers for bacteria or microorganisms and/or as part of a flow cell.
